Europäisches Patentamt

European Patent Office

Office européen des brevets

(11) Publication number: **0 354 599**
**A1**

# EUROPEAN PATENT APPLICATION

(21) Application number: **89200292.4**

(22) Date of filing: **08.02.89**

(51) Int. Cl.⁴: **A61B 17/58 , A61B 17/064**

(30) Priority: **03.08.88 IT 5334788 U**

(43) Date of publication of application:
**14.02.90 Bulletin 90/07**

(84) Designated Contracting States:
**AT BE CH DE ES FR GB LI NL SE**

(71) Applicant: **D.A.O. s.r.l.**
**Corso Matteotti No. 30**
**I-10121 Turin(IT)**

(72) Inventor: **Del Medico, Nilli**
**Via Nazario Sauro 34**
**I-10043 Orbassano (Turin)(IT)**

(74) Representative: **Robba, Eugenio et al**
**Studio "INTERPATENT" via Caboto 35**
**I-10129 Turin(IT)**

(54) **An adjustable staple.**

(57) An adjustable staple to be used in bone surgery, comprising a horizontally laying U-shaped member wherein the lower arm (3) is movable parallel to itself in respect of the upper fixed arm (1), and adapted to be fastened by pressure in any position; said movement taking place by means of a dovetail joint between the jportion (5) orthogonal to the upper arm (1) and the portion (7) orthogonal to the lower arm (3); the fastening once reached the desired position being provided by two socket head screws (9), aligned and opposed, engaging corresponding seats in the portion (5) of the movable arm and acting with pressure against the portion (7) of the movable arm.

FIG.1

EP 0 354 599 A1

# AN ADJUSTABLE STAPLE

The present invention relates to an adjustable span staple, particularly useful in the technique of bone surgery.

It is known that until presently, for example in order to straighten a bone, the usual practice is to carry out an osteotomy of such bone, consisting of a more or less deep transverse wedge cut of the concerned bone, a correct positioning of the concerned portion of such bone by drawing together the two faces of the cut until they match, then fixing the two portions, above and below the osteotomy, by means of a staple, and finally plastering the limb until the bone structure has knilted.

A typical situation is a crooked leg, that is where the shinbone has to be straighten out by aligning it with the thighbone; in such a case the osteotomy is carried out on the shinbone, the lower portion of the shinbone, below the cut, is straightened out and correctly positioned, and then fastened in such position through a staple which is driven into the shinbone above and below the osteotomy, thus maintaining the two bone portions in proper relationship; thereafter, as stated above, a plaster also covering the thighbone is applied to the limb until the bone recovery.

It is clear that in such a situation the limb, a leg in the present case, is to be immobilized for at least forty days and the knee joint cannot be used, neither the patient can rest on the footh.

In the known art, such as the above mentioned case of a shinbone operation, the employed staples have fixed spans, i.e. the distance between the upper arm and the lower arm is fixed and predetermined for each type of staple, so that a large number of these staples are required in a hospital since the cuts are different each times as for what concerns the extension, the dept and so on.

The object of the present invention is to overcome the above shortcomings by providing an adjustably spanning staple which besides the consistent advantage of drastically reducing the number of staples required in a hospital or orthopedic clinic, provides for the outstanding advantage of avoiding the plastering after the surgical operation, a post-operative bandaging being enough, so that the patient can immediately move the limb joint, e.g. the knee joint, and as a matter of fact is able to rest on the leg one or two days after the operation.

Thus the surgeon's task is greatly helped and the surgical operation is rendered quicker and more practical, as well as less annoying for the patient no longer forced to immobility.

The present adjustable staple comprises a horizontally laying U-shaped member wherein the upper tab of the U forms the upper arm of the staple, the lower tab the lower arm of the same, and the connecting segment of the two tabs (i.e. the bottom of the U) the portion orthogonal to the two arms, and is characterized in that the lower arm of the staple is movable parallel to itself in respect of the upper fixed arm, and is adapted to be fastened by pressure in any position along said movement; said movement taking place by means of a dovetail joint between the portion orthogonal to the upper arm and the portion orthogonal to the lower arm; said movement of the portions being manually carried out by the operator; the fastening once reached the desired position being provided by two socket head screws engaging corresponding aligned and opposed threaded seats in the portion of the fixed arm, and exerting a pressure against the portion of the movable arm.

An additional characteristic of the present invention is that the socket head screws for fastening the movable member with respect to the fixed member provide for a polygonal socket, e.g. hexagonal, so as to be easily driven by a control rod having the same polygonal cross section, e.g. hexagonal.

A further characteristic of the present invention resides in that the staple movable arm can be fitted on the portion pertaining to the fixed arm in a reversed position, that is in such a way as to present the movable arm in a decidedly nearer position to the parallel fixed arm, to allow for, as a limit, the contact between the two arms of the staple.

The invention will now be disclosed with particular reference to the attached drawings, illustrating a nonlimiting embodiment, in which:

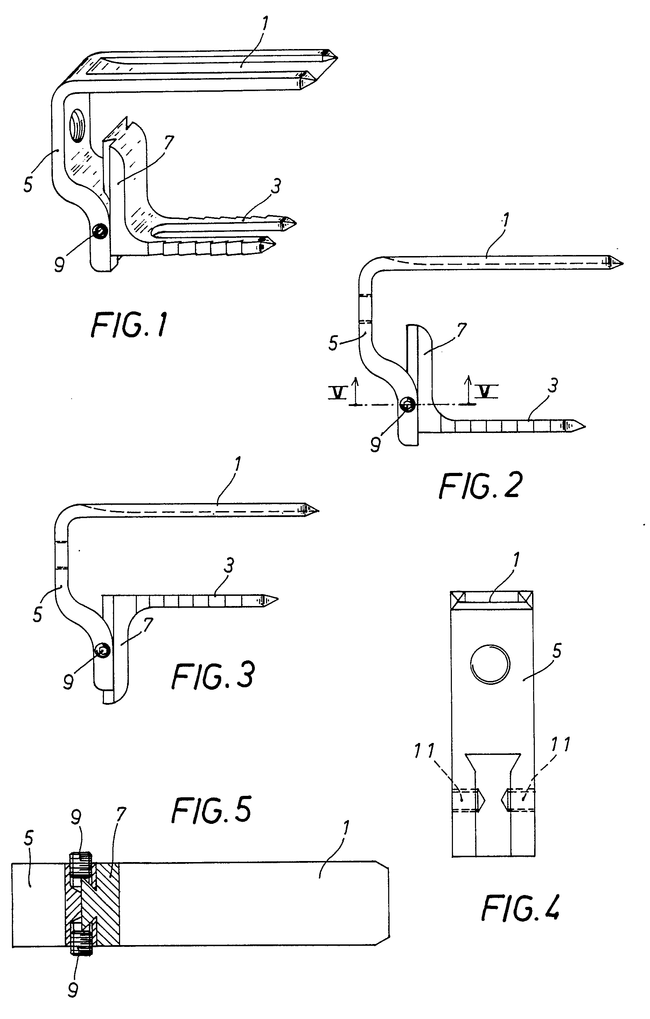

- Fig. 1 is a perspective view of the staple of the invention with the movable arm slightly raised;

- Fig. 2 is an elevation side view of the staple of Fig. 1;

- Fig. 3 is an elevation side view of the staple of Fig. 1 with the movable arm overturned;

- Fig. 4 is a front view of the upper (fixed) arm of the staple;

- Fig. 5 is a cross section along line V-V of Fig. 2.

As clearly illustrated in the Figures, the staple of the invention is substantially similar to the conventional staples with a fixed distance between the arms, of the type usually employed in bone surgery.

It is substantially formed as a horizontally laying U-shaped member, the upper tab of the U forming the upper arm, the lower tab the lower arm, and the the connecting segment of said two tabs (the bottom of the U) the portion orthogonal to the said two arms.

According to the invention, the lower arm 3 is movable in respect of the fixed arm 1, the relative displacement between said movable arm 3 and the fixed arm 1 taking place along a direction which is parallel to the portion 5 connecting the two arms 1 and 3.

More particularly, on portion 5 there is provided a dovetail coupling or joint between the portion 5 pertaining to the fixed arm, and the portion 7 pertaining to the movable arm 3. Said two portions 5 and 7 can therefore slide in respect of each other shifting the movable arm 3 parallel to itself as desired with respect to the fixed arm.

Said shifting is manually accomplished by the operator.

Once the desired position has been reached, the fastening of the movable arm 3 with respect to the fixed arm 1 is obtained by means of two socket head screws 9 fitting at 11 into the portion 5 pertaining to the fixed arm 1, which are aligned and opposed and adapted, when tightened, to exert a pressure against the dovetail member of the portion 7 pertaining to the movable arm 3 when tightened; with the tightening of said screw 9 and the consequent pressure over the portion 7 determining the relative position of the two arms.

In the illustrated embodiment, the socket head screws 9 provided for hexagonal sockets (Allen screws); a suitable wrench having an equal polygonal cross section, hexagonal in this case, will be used for turning them in a direction (tightening) and in the opposite direction (loosening).

It is to be noted that the movable arm 3 of the staple can be mounted on the portion 5 pertaining to the fixed arm 1 in a reversed position, such as to position the movable arm 3 in a position decidedly nearer to the parallel fixed arm 1, to allow for, as a limit, the contact between the two arms of the staple.

## Claims

1. An adjustable staple to be used in bone surgery, comprising a horizontally laying U-shaped member wherein the upper tab of the U forms the upper arm (1) of the staple, the lower tab the lower arm (3) of the same, and the connecting segment of the two tabs (i.e. the botton of the U) the portion orthogonal to the two arms, characterized in that the lower arm (3) of the staple is movable parallel to itself in respect of the upper fixed arm (1), and is adapted to be fastened by pressure in any position along said movement; said movement taking place by means of a dovetail joint between the portion (5) orthogonal to the upper arm (1) and the portion (7) orthogonal to the lower arm (3); said movement of the portions (5, 7) being manually carried out by the operator; the fastening once reached the desired position being provided by two socket head screws (9) engaging corresponding aligned and opposed threaded seats in the portion (5) of the fixed member (1), and exerting a pressure against the portion (7) of the movable arm (3).

2. An adjustable staple as claimed in claim 1, characterized in that the socket head screws (9) for fastening the movable member (3) with respect to the fixed member (1) provide for a polygonal socket, e.g. hexagonal, so as to be driven by a control rod having the same polygonal cross section, e.g. hexagonal.

3. An adjustable staple as claimed in claim 1, characterized in that the staple movable arm (3) can be fitted on the portion (5) pertaining to the fixed arm (1) in a reversed position, that is in such a way as to present the movable arm (3) in a decidedly nearer position to the parallel fixed arm (1), to allow for, as a limit, the contact between the two arms (1, 3) of the staple.

EP 0 354 599 A1

FIG.1

FIG.2

FIG.3

FIG.5

FIG.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. '5) |
|---|---|---|---|
| A | US-A-4 723 540  (GILMER)<br>* Abstract; figures *<br>--- | 1 | A 61 B  17/58<br>A 61 B  17/064 |
| A | GB-A-1 350 981  (AUSTIN)<br>--- | | |
| A | CH-A- 335 797  (ULRICH & CO.)<br>--- | | |
| P,A | EP-A-0 301 898  (OUTERBRIDGE)<br>* Figures 1-12; column 3, line 45 - column 7, line 37 *<br>----- | 1,3 | |
| | | | TECHNICAL FIELDS SEARCHED (Int. Cl. 5)<br><br>A 61 B |

The present search report has been drawn up for all claims

| Place of search | Date of completion of the search | Examiner |
|---|---|---|
| THE HAGUE | 05-11-1989 | STEENBAKKER J. |

EPO FORM 1503 03.82 (P0401)